# FASCICULE DE BREVET EUROPEEN

(11) **EP 3 829 762 B1**
(45) Date de publication et mention de la délivrance du brevet: **07.08.2024**
(21) Numéro de dépôt: 19737741.9
(22) Date de dépôt: 10.07.2019
(51) Int. Cl.: B01J 20/10, B01J 20/22, B01J 20/28, B01J 20/283, B01J 20/30, B01D 53/04, A61L 9/014, A61L 9/04, B01D 53/02

(54) **PROCÉDÉ DE PRÉPARATION DES MATÉRIAUX SOL-GEL ADSORBANT DES ALDÉHYDES ET CÉTONES**
HERSTELLUNGSVERFAHREN FÜR SOL-GEL-MATERIAL, DAS DIE ALDEHYDE UND KETONE ADSORBIERT
METHOD FOR PREPARING SOL-GEL MATERIAL ADSORBING ALDEHYDES AND KETONES

(30) Priorité: 27.07.2018 EP 18186191; 28.01.2019 EP 19154042
(43) Date de publication de la demande: 09.06.2021
(73) Titulaire: SEB S.A., 69130 Ecully (FR)
(72) Inventeur: GINEYS, Mickael, 38350 LA MURE (FR); HAMMEL, Frederic, 13600 LA CIOTAT (FR); PACCAUD, Denis, 69008 LYON (FR); SIBEAUD, Mathilde, 69009 LYON (FR); SOM, Marie-Pierre, 26120 MONTELIER (FR)
(74) Mandataire: SEB Développement
(86) Numéro de dépôt international: PCT/EP2019/068512
(87) Numéro de publication internationale: WO 2020/020639

(56) Documents cités:
- EP-A1- 2 615 125
- EP-A1- 3 045 224
- WO-A1-2016/046498
- WO-A1-2016/167258
- JP-A- 2010 235 363
- JP-A- 2017 160 356

## Description

L'invention se rapporte à un procédé de préparation d'un matériau sol-gel adsorbant les aldéhydes et les cétones.

Les aldéhydes comptent parmi les polluants chimiques domestiques les plus abondants. Leurs sources sont extrêmement nombreuses. Elles peuvent être notamment en relation avec une production extérieure comme la photo-oxydation du méthane. Cependant, les sources principales d'émission des aldéhydes se trouvent à l'intérieur des habitations et sont très diverses : les résines et les colles servant à fabriquer les bois agglomérés, les panneaux de particules et les contreplaqués, les revêtements textiles, papiers peints, peintures, cuirs, les mousses isolantes urée-formol utilisées comme isolants thermiques par injection dans les murs et les cloisons.

Le formaldéhyde est également un agent conservateur, désinfectant et déshydratant. Pour ces raisons, il est abondamment employé comme solvant en milieu hospitalier pour la désinfection des instruments chirurgicaux mais aussi dans l'industrie des services funéraires où l'on pratique la thanatopraxie.

Compte tenu des effets nocifs de tels polluants chimiques sur la santé publique, il apparaît nécessaire d'assurer la purification de l'air ambiant des bâtiments d'habitation en réduisant la teneur en aldéhydes, et notamment en formaldéhyde, et en offrant de nouveaux dispositifs de dépollution.

Il est connu de traiter l'air et de le purifier par adsorption par des matériaux adsorbants. Il s'agit d'un phénomène de surface par lequel les polluants de l'air tels que des atomes, des ions ou des molécules (adsorbats) se fixent sur la surface solide du matériau adsorbant. Cette fixation peut être :
- soit physique mettant en jeu des liaisons faibles, du type forces de van der Waals entre les espèces chimiques adsorbées et l'adsorbant,
- soit chimique mettant en jeu des énergies de liaison importantes, du type liaisons covalentes, ioniques ou métalliques entre les espèces chimiques adsorbées et l'adsorbant.

Le phénomène inverse, par lequel les molécules adsorbées sur une surface s'en détachent, par exemple sous l'action de l'élévation de la température, ou de la baisse de pression, est la désorption.

Il est possible de rendre adsorbant un matériau par fixation sur ce matériau d'une molécule sonde capable de piéger le polluant et de le retenir sans le dégrader. Le matériau est généralement poreux, de préférence microporeux, plus préférentiellement nanoporeux, avec une grande surface spécifique. La molécule sonde est généralement déposée par imprégnation sur le matériau afin de faciliter les phénomènes de surface dans la structure poreuse du matériau. Selon le document FR 2890745, il est connu un matériau poreux pour l'élimination des aldéhydes et plus précisément du formaldéhyde qui est une matrice nanoporeuse d'oxydes métalliques comportant au moins une fonction réactive aux aldéhydes. Les molécules sondes décrites sont les énaminones et les couples 3-dicétone/amine correspondants, les imines et les hydrazines, ou les sels dérivés de ces composés.

Le document EP 2 615 125 A1 est un procédé de préparation d'un adsorbant de matériau sol-gel.

Cependant, il a été constaté que ces matériaux présentent une baisse de leur résistance mécanique et une migration de la molécule sonde en atmosphère humide, voire tropicale. Ainsi en atmosphère humide (environ 60-80% d'humidité relative (HR) voire plus), ces matériaux deviennent friables et s'effritent. De plus, la molécule sonde a tendance à s'échapper du matériau poreux et à être relarguée dans son environnement, le matériau perdant ses capacités de piégeage des polluants.

Aussi, la présente invention a pour but de résoudre tout ou partie des inconvénients mentionnés ci-dessus, notamment en proposant un procédé de préparation d'un matériau poreux adsorbant présentant une résistance mécanique améliorée et une absence de migration de la molécule sonde en atmosphère tropicale (par exemple à environ 80% d'humidité relative), tout en présentant avantageusement une quantité réduite de molécule sonde dans le matériau.

De manière inattendue, les inventeurs ont mis en évidence qu'il est possible d'utiliser certains acides organiques et certaines molécules sondes pour fabriquer un matériaux sol-gel nanoporeux adsorbants les aldéhydes et les cétones évitants les inconvénients ci-dessus.

L'invention offre au moins un des avantages déterminants décrits ci-après :
- piéger de manière définitive et irréversible les polluants de l'air ambiant par adsorption de polluants ; alors qu'avec des matériaux adsorbants classiques tels que notamment les zéolites ou le charbon actif, les polluants sont susceptibles d'être relargués;
- fixer la molécule sonde dans le matériau sol-gel et éviter la migration dans l'air ambiant de cette molécule sonde, en particulier en atmosphère tropicale ou de forte humidité (comme par exemple à environ 60- 80% d'humidité relative);
- améliorer la résistance mécanique du matériau dans des conditions tropicales ou de fortes humidité ; et
- présenter un matériau avec une quantité réduite de molécule sonde fixée dans le matériau à performance de piégeage comparable.

D'autres avantages et caractéristiques de l'invention apparaîtront clairement à la lecture de la description et des exemples donnés à titre purement illustratifs et non limitatifs qui vont suivre.

A cet effet, l'invention se rapporte à un procédé tel que défini dans la revendication 1 pour la préparation d'un matériau sol-gel poreux adsorbant comprenant les étapes successives suivantes :
i) préparer une composition aqueuse comprenant au moins
   - un précurseur sol-gel de type alcoxysilane ;
   - un acide choisi parmi l'acide sulfamique, l'acide phosphorique, l'acide paratoluène sulfonique, l'acide parahydrobenzoique et leurs mélanges;
   - une molécule sonde de formule générale (I) ou un de ses sels

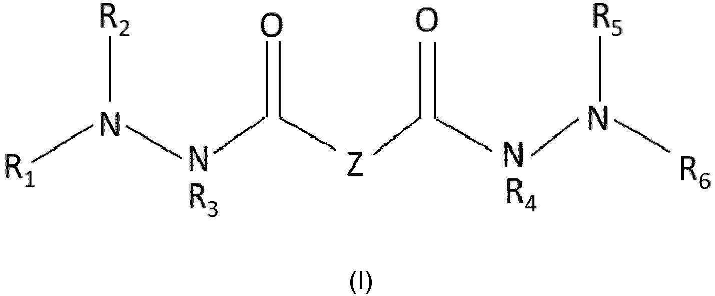

   avec R₁, R₂, R₃, R₄, R₅, R₆ représentant indépendamment un atome d'hydrogène, un groupement (C1-C6)alkyle, un groupement (C3-C7)cycloalkyle ou un groupement alkyl-(C3-C7)cycloalkyle ;
   avec Z représentant un groupement espaceur choisi parmi un groupement (C1-C16)alkyle, un groupement (C2-C16)alcényle, un groupement (C2-C16)alcynyle, un groupement (C1-C16)halogénoalkyle, un groupement aryle, un groupement aryloxy, un groupement carbocycle et un groupement aryl-(C1-C16)alkyle ;
ii) mettre en forme la composition obtenue à l'étape i) et appliquer un traitement thermique à une température supérieure à 20°C pour que la réaction d'hydrolyse / condensation se produise;
iii) sécher le matériau sol-gel adsorbant obtenu.

Généralement, un procédé sol-gel utilise comme précurseurs des alcoxydes métalliques de formule M(OR)xR'n-x où M est un métal, notamment le silicium, R un groupement alkyle et R' un groupement porteur d'une ou de plusieurs fonctions avec n= 4 et x pouvant varier entre 2 et 4. En présence d'eau, les groupements alkoxy (OR) sont hydrolysés en groupements silanols (Si-OH). Ces derniers se condensent en formant des liaisons siloxanes (Si-O-Si-). C'est la réaction d'hydrolyse / condensation.

Le procédé de préparation d'un matériau sol-gel poreux adsorbant selon l'invention met en oeuvre un précurseur sol-gel de type alcoxysilane. De préférence, ce précurseur sol-gel de type alcoxysilane est choisi parmi l'orthosilicate de tétraméthyle (TMOS), le méthyltriméthoxysilane (MTMS), le tétraéthoxysilane (TEOS), le méthyltriéthoxysilane (MTES), le diméthyldiméthoxysilane et leurs mélanges, de préférence l'orthosilicate de tétraméthyle aussi appelé tétraméthoxysilane.

La synthèse du gel à l'étape i) est avantageusement réalisée à partir de tétraméthoxysilane ou d'un mélange de tétraméthoxysilane et d'au moins un autre précurseur organosilicé choisi parmi le phényltriméthoxysilane, le phényltriéthoxysilane, le fluoroalkyltriméthoxysilane, le fluoroalkyltriéthoxysilane, le chloroalkyltriméthoxysilane, le chloroalkyltriéthoxysilane, le alkyltriméthoxysilane, un alkyltriéthoxysilane, le aminopropyltriéthoxysilane et leurs mélanges.

De préférence la synthèse du gel à l'étape i) est réalisée à partir de tétraméthoxysilane ou d'un mélange de tétraméthoxysilane et d'au moins un autre précurseur organosilicé choisi parmi le chloroalkyltriméthoxysilane, le chloroalkyltriéthoxysilane, le aminopropyltriéthoxysilane et leurs mélanges.

Préférentiellement, la synthèse du gel à l'étape i) est réalisée à partir de tétraméthoxysilane ou d'un mélange de tétraméthoxysilane et d'au moins un autre précurseur organosilicé choisi parmi le chloropropyltriméthoxysilane, le chloropropyltriéthoxysilane, le (C3-C10)alkyletriméthoxysilane, le (C3-C10)alkyletriéthoxysilane, le aminopropyltriéthoxysilane et leurs mélanges.

Plus préférentiellement, la synthèse du gel à l'étape i) est réalisée à partir de tétraméthoxysilane ou d'un mélange de tétraméthoxysilane et d'au moins un autre précurseur organosilicé choisi parmi le propyltriméthoxysilane, le propyltriéthoxysilane, le chloropropyltriméthoxysilane, le chloropropyltriéthoxysilane, le propyltriméthoxysilane, le propyltriéthoxysilane, le aminopropyltriéthoxysilane et leurs mélanges.

Plus préférentiellement la synthèse du gel à l'étape i) est réalisée à partir de tétraméthoxysilane ou d'un mélange de tétraméthoxysilane et d'au moins un autre précurseur organosilicé choisi parmi le chloropropyltriméthoxysilane et un aminopropyltriéthoxysilane, plus préférentiellement encore choisi parmi le (3-chloropropyl)triméthoxysilane (CITMOS), et le (3-aminopropyl)triéthoxysilane (APTES).

Plus préférentiellement encore l'étape i) est réalisée à partir de tétraméthoxysilane ou d'un mélange de tétraméthoxysilane et de (3-chloropropyl)triméthoxysilane (CITMOS), ou d'un mélange de tétraméthoxysilane et de (3-aminopropyl)triéthoxysilane (APTES).

Le procédé de préparation d'un matériau sol-gel poreux adsorbant selon l'invention met en oeuvre une molécule sonde de formule générale (I) ou un de ses sels.

Par groupement « (C1-C6)alkyle », il faut comprendre, au sens de la présente invention, une chaîne hydrocarbonée monovalente saturée, linéaire ou ramifiée, comportant 1 à 6, de préférence 1 à 4, atomes de carbone. A titre d'exemple, on peut citer les groupes méthyle, éthyle, propyle, isopropyle, butyle, isobutyle, sec-butyle, tert-butyle, pentyle ou encore hexyle.

Par groupement « (C3-C7)cycloalkyle », il faut comprendre, au sens de la présente invention, une chaîne hydrocarbonée saturée cyclique, comportant 3 à 7 atomes de carbone cycliques. A titre d'exemple, on peut citer les groupes cyclopropyle, cyclopentyle, cyclohexyle et cycloheptyle.

Par « alkyl-(C3-C7)cycloalkyle », il faut comprendre, au sens de la présente invention, un groupe (C3-C7)cycloalkyle tel que défini ci-dessus, lié au reste de la molécule par l'intermédiaire d'une chaîne (C1-C6)alkyle telle que définie ci-dessus.

Par groupement « (C1-C16)alkyle », il faut comprendre, au sens de la présente invention, une chaîne hydrocarbonée monovalente saturée, linéaire ou ramifiée, comportant 1 à 16, de préférence 1 à 10, plus préférentiellement de 1 à 8 atomes de carbone.

Par groupement « (C2-C16)alcényle », il faut comprendre, au sens de la présente invention, une chaîne hydrocarbonée monovalente, linéaire ou ramifiée, comportant au moins une double liaison et comportant 2 à 16 atomes de carbone. A titre d'exemple, on peut citer les groupes éthényle ou allyle.

Par groupement « (C2-C16)alcynyle », il faut comprendre, au sens de la présente invention, une chaîne hydrocarbonée monovalente, linéaire ou ramifiée, comportant au moins une triple liaison et comportant 2 à 16 atomes de carbone. A titre d'exemple, on peut citer les groupes éthynyle ou propynyle.

Par « (C1-C16)halogénoalkyle », il faut comprendre, au sens de la présente invention, un groupe (C1-C16)alkyle, tel que défini ci-dessus, pour lequel un ou plusieurs atomes d'hydrogène ont été remplacés par un atome d'halogène tel que défini ci-dessous. Il peut s'agir en particulier d'un groupe CF3.

Par « atome d'halogène », il faut comprendre, au sens de la présente invention, les atomes de fluor, de chlore, de brome ou d'iode.

Par « aryle », il faut comprendre, au sens de la présente invention, un groupement hydrocarboné aromatique, comportant de préférence de 6 à 10 atomes de carbone, et comprenant un cycle ou plusieurs cycles accolés, comme par exemple un groupement phényle ou naphtyle. Avantageusement, il s'agit du phényle.

Par le terme « groupe aryloxy », il faut comprendre, au sens de la présente invention, tout groupe aryle tel que défini ci-dessus, lié au reste de la molécule par l'intermédiaire d'au moins un atome d'oxygène. Il peut s'agir en particulier d'un groupement phényloxy.

Par « carbocycle », il faut comprendre, au sens de la présente invention, un système monocyclique ou polycyclique hydrocarboné, saturé, insaturé ou aromatique, comprenant de 3 à 12 atomes de carbone. Le système polycyclique comprend au moins 2, notamment 2 ou 3, cycles accolés ou pontés. Chaque cycle du système monocyclique ou polycyclique comprend avantageusement 3 à 8, notamment 4 à 7, en particulier 5 ou 6, atomes de carbone. A titre d'exemple on peut citer un groupe adamantyle, cyclohexyle, cyclopentyle, cyclopropyle, cyclohexényle, phényle, naphtyle.

Par « aryl-(C1-C6)alkyle », il faut comprendre, au sens de la présente invention, un groupe aryle tel que défini ci-dessus, lié au reste de la molécule par l'intermédiaire d'une chaîne (C1-C6)alkyle telle que définie ci-dessus. A titre d'exemple, on peut citer le groupe benzyle.

De préférence, le procédé de préparation d'un matériau sol-gel poreux adsorbant selon l'invention met en oeuvre une molécule sonde de formule générale (I) avec R₁, R₂, R₃, R₄, R₅, R₆ représentant un atome d'hydrogène ou un groupement (C1-C6)alkyle.

De préférence, le procédé de préparation d'un matériau sol-gel poreux adsorbant selon l'invention met en oeuvre une molécule sonde de formule générale (I) avec Z représentant un groupement (C1-C16)alkyle, de préférence un groupement (C1-C10)alkyle, ou un groupe alkyle en C4, C6 ou C8.

De préférence, le procédé de préparation d'un matériau sol-gel poreux adsorbant selon l'invention met en oeuvre une molécule sonde de formule générale (I) avec Z représentant un groupement aryle ou un groupement aryl-(C1-C6)alkyle.

De préférence, le procédé de préparation d'un matériau sol-gel poreux adsorbant selon l'invention met en oeuvre une molécule sonde de formule générale (I) avec R₁, R₂, R₃, R₄, R₅, R₆ représentant un atome d'hydrogène ou un groupement (C1-C6)alkyle, et avec Z représentant un groupement (C1-C16)alkyle, de préférence un groupement (C1-C10)alkyle, ou un groupe alkyle en C4, C6 ou C8.

De préférence, le procédé de préparation d'un matériau sol-gel poreux adsorbant selon l'invention met en oeuvre une molécule sonde de formule générale (I) avec R₁, R₂, R₃, R₄, R₅, R₆ représentant un atome d'hydrogène ou un groupement (C1-C6)alkyle, et avec Z représentant un groupement aryle ou un groupement aryl-(C1-C6)alkyle.

Selon l'invention, il est possible de combiner les différents groupements de la molécule sonde de formule générale (I).

De préférence, le procédé de préparation d'un matériau sol-gel poreux adsorbant selon l'invention met en oeuvre une molécule sonde ou son sel qui est
- le dihydrazide d'acide adipique (C₆H₁₄N₄O₂), 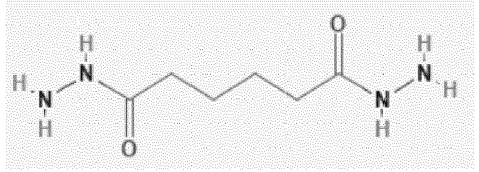
- le dihydrazide d'acide sébacique (C₁₀H₂₂N₄O₂) 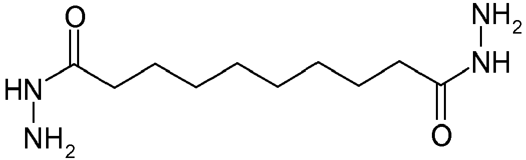
- le dihydrazide de l'acide téréphtalique (C₈H₁₀N₄O₂).

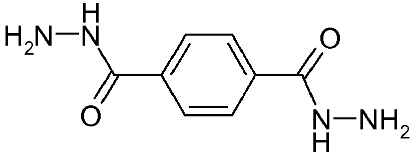

La fonction amine de la molécule sonde étant un groupement fonctionnel basique, celle-ci peut se présenter sous forme de sels. Ces sels comprennent notamment les sels d'addition d'acide de la molécule sonde.

Le procédé de fabrication du matériau sol-gel poreux adsorbant selon l'invention permet une introduction de la molécule sonde lors de son élaboration, c'est-à-dire in-situ ou One-pot (en anglais) et non pas par imprégnation, c'est-à-dire non pas par mise en contact d'un liquide avec un solide ou semi solide. Il s'agit avantageusement d'une synthèse du gel à l'étape i) du type synthèse monotope. La synthèse monotope ou synthèse one-pot est généralement une synthèse chimique dans laquelle un réactif subit plusieurs réactions dans un seul mélange réactionnel (un seul réacteur, par exemple). C'est-à-dire que la synthèse est réalisée en une seule étape avec un précurseur sol-gel de type alcoxysilane, par exemple le tétraméthoxysilane ou un mélange de tétraméthoxysilane et un autre précurseur organosilicé, et le ou les molécules sondes en présence de l'acide inorganique et/ou organique et d'eau et éventuellement d'un solvant organique polaire. Cela signifie qu'il n'y a pas d'imprégnation de la molécule sonde sur le matériau sol gel, après que la fabrication de celui-ci soit terminée, mais une combinaison intime des matériaux lors de la préparation du sol-gel. Avantageusement, la combinaison de la molécule sonde avec un précurseur sol-gel de type alcoxysilane se fait à l'état liquide ou semi liquide.

Avantageusement, le procédé selon l'invention avec sa synthèse monotope permet de gagner du temps et d'augmenter le rendement chimique global.

Le procédé de fabrication du matériau sol-gel poreux adsorbant selon l'invention permet avantageusement de réduire les quantités de molécules sondes comparé aux procédés par imprégnation. La saturation de la surface spécifique par les méthodes par imprégnation est ainsi évitée.

Il est à noter que le procédé de préparation d'un matériau sol-gel poreux adsorbant selon l'invention n'est pas un procédé par imprégnation ou immersion de la molécule sonde.

Dans un mode de réalisation, le matériau sol-gel poreux adsorbant selon l'invention ne contient ni zéolite ni charbon actif.

Lors de l'utilisation d'un mélange de tétraméthoxysilane et d'un autre précurseur organosilicé, les proportions molaires de tétraméthoxysilane/autre précurseur organosilicé peuvent varier de 1/0,01 à 1/0,2, de préférence de 1/0,01 à 1/0,04.

La composition aqueuse mise en oeuvre à l'étape i) du procédé selon l'invention comprend un ratio molaire précurseur d'alcoxysilane / molécule sonde compris de 1/0,1 à 1/0,001, de préférence de 1/0,08 à 1/0,002, plus préférentiellement de 1/0,075 à 1/0,010, et en particulier un ratio égal à 1/0,05 ou 1/0,015.

La composition aqueuse mise en oeuvre à l'étape i) du procédé selon l'invention comprend un ratio molaire de TMOS / molécule sonde compris de 1/0,1 à 1/0,001, de préférence de 1/0,08 à 1/0,002, plus préférentiellement de 1/0,075 à 1/0,010, et en particulier un ratio égal à 1/0,05 ou 1/0,015.

La synthèse du gel à l'étape i) est avantageusement réalisée en milieu aqueux en présence d'un acide inorganique et/ou organique dont la température d'ébullition est supérieure ou égale à 100°C, de préférence supérieure ou égale à 150°C, plus préférentiellement supérieure ou égale à 180°C. En particulier, l'acide mis en oeuvre selon le procédé de l'invention est un acide présentant une non volatilité importante.

La synthèse du gel à l'étape i) est avantageusement réalisée en milieu aqueux en présence d'un acide de préférence un acide ayant un pKA d'au plus 3,5, de préférence d'au plus 2,8.

Selon l'invention l'acide est choisi parmi l'acide sulfamique, l'acide phosphorique, l'acide paratoluène sulfonique, l'acide parahydroxybenzoique et leurs mélanges.

En particulier, l'acide mis en oeuvre selon le procédé de l'invention est un acide faiblement hygroscopique.

La réalisation de la synthèse en milieu acide permet d'obtenir de meilleures performances de coloration de la matrice sol-gel lorsque la molécule sonde, et en particulier son groupement amine, réagit avec des aldéhydes et/ou cétones. De bonnes performances sont obtenues lorsque le pH du milieu aqueux est inférieur à 3, de préférence inférieur à 2, préférentiellement inférieur à 2,5, plus préférentiellement inférieur à 2,2 et encore plus préférentiellement inférieur à 1,8.

La synthèse du gel à l'étape i) est réalisée en milieu aqueux. Avantageusement, le milieu aqueux est de l'eau ou un mélange d'eau et éventuellement d'un solvant organique polaire. Le solvant organique polaire peut être un solvant organique protique, de préférence un alcool aliphatique en C1 à C6, plus préférentiellement du méthanol ou de l'éthanol. L'homme du métier saura déterminer facilement les quantités nécessaires d'eau et éventuellement de solvant organique polaire en fonction du ou des précurseurs organosilicés employés.

Le procédé comprend une étape ii) de mise en forme de la composition aqueuse obtenue à l'étape i). Il s'agit par exemple de déposer ou verser cette composition, de préférence liquide ou semi liquide dans un contenant qui lui donnera sa forme.

Le procédé comprend une étape iii) de séchage du matériau sol-gel obtenu après l'étape ii). Il s'agit d'une étape d'évaporation de l'eau contenue dans le matériau. Celle-ci peut avoir lieu à température ambiante pendant une durée prolongée de quelques jours et sous l'action de la chaleur ou non.

Selon une variante du procédé selon l'invention, celui-ci comprend une étape iv) de cuisson du matériau sol-gel en sus de l'étape iii) de séchage. Cette étape additionnelle de cuisson du matériau adsorbant sol-gel après sa réalisation peut avoir lieu à une température comprise de 50 à 150°C, de préférence de 60 à 80°C et en particulier à 70°C.

Il est également décrit, mais non revendiqué, un matériau sol-gel poreux adsorbant comprenant au moins
- des oxydes de silane;
- un acide inorganique et/ou organique dont la température d'ébullition est supérieure à 100°C;
- une molécule sonde de formule générale (I) suivante

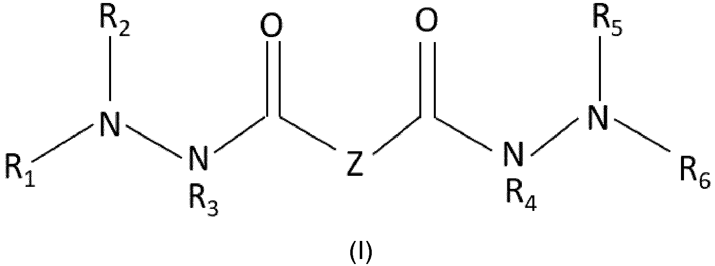

avec R₁, R₂, R₃, R₄, R₅, R₆ représentant indépendamment un atome d'hydrogène, un groupement (C1-C6)alkyle, un groupement (C3-C7)cycloalkyle ou un groupement alkyl-(C3-C7)cycloalkyle ;
avec Z représentant un groupement espaceur choisi parmi un groupement (C1-C16)alkyle, un groupement (C2-C16)alcényle, un groupement (C2-C16)alcynyle, un groupement (C1-C16)halogénoalkyle, un groupement aryle, un groupement aryloxy, un groupement carbocycle et un groupement aryl-(C1-C16)alkyle.

Ce qui a été décrit plus haut concernant le procédé selon l'invention et relatif aux acides et molécules sondes s'applique de la même manière concernant le matériau De même, ce qui a été décrit plus haut concernant le précurseur sol-gel de type alcoxysilane s'applique de la même manière pour l'oxyde de silane du matériau

Dans la suite du texte, il est indistinctement employé l'expression matériau pour se référer au matériau sol-gel poreux adsorbant selon l'invention ou au matériau susceptible d'être obtenu par le procédé selon l'invention. Le matériau est également appelé matrices sol-gel silicatés, c'est-à-dire des matrices sol-gels obtenues à partir d'alcoxydes métalliques de formule M(OR)xR'n-x où M est Si.

Les oxydes de silane du matériau sont ceux obtenus après réaction d'hydrolyse/condensation de précurseurs sol-gel de type alcoxysilane, mis en oeuvre dans le procédé selon l'invention et décrits plus haut. Les oxydes de silane du matériau sont généralement des polysiloxanes, obtenus après réaction d'hydrolyse/condensation de précurseurs sol-gel de type alcoxysilane

Le matériau sol-gel poreux adsorbant peut se présenter sous la forme de granulés de forme cylindrique avec un rapport L/D>1 dans lequel L est la longueur du granulé et D est le diamètre du granulé.

Il est également décrit, mais non revendiqué l'utilisation d'un matériau susceptible d'être obtenu par le procédé selon l'invention pour capturer des aldéhydes et/ou des cétones, en particulier des aldéhydes choisis parmi le formaldéhyde, l'acétaldéhyde, le propionaldéhyde, le butyraldéhyde, l'acroléine, le pentanal, l'hexanal, le benzaldéhyde et leur mélange.

Par aldéhyde, on désigne toute molécule organique ayant une fonction carbonyle terminale choisie de préférence parmi le formaldéhyde, l'acétaldéhyde, le propionaldéhyde, le butyraldéhyde, l'acroléine, le pentanal, l'hexanal et le benzaldéhyde.

Il est également décrit, mais non revendiqué un appareil de purification de l'air mettant en oeuvre un matériau susceptible d'être obtenu par le procédé selon l'invention.

Lors du passage de l'air pollué dans le matériau sol-gel poreux adsorbant le polluant réagit avec la molécule sonde pour former une troisième entité chimique d'un poids moléculaire plus important qui restera piégée dans le réseau nanoporeux du média filtrant comprenant un matériau adsorbant spécifique. Contrairement aux autres adsorbants, ce matériau adsorbant spécifique effectue un piégeage définitif par transformation chimique irréversible du polluant. Sans être lié par aucune théorie, lorsque par exemple un aldéhyde ou une cétone entre en contact avec le ou les groupements aminés de la molécule sonde, le groupement carbonyle réagit avec le groupement amine pour donner un groupement imine. De manière générale, cette première réaction ne produit pas de coloration. En présence d'une forte concentration de composés aminés et de polluants dans les pores, cela peut induire d'autres réactions qui conduisent à un produit coloré. A titre d'exemple, il est obtenu à saturation une coloration jaune-orange lorsque la matrice selon l'invention est exposée à du formaldéhyde ou de l'acétone et une coloration rouge-brun avec le benzaldéhyde.

Lorsque les polluants sont de taille élevée comme le benzaldéhyde, la porosité du matériau sol-gel permet une diffusion rapide des aldéhydes/cétones dans la matrice afin de les piéger et les faire réagir avec les groupements aminés de la molécule sonde et obtenir un changement de coloration à saturation.

Le matériau est généralement poreux, de préférence microporeux, plus préférentiellement nanoporeux ou mésoporeux, avec une grande surface spécifique.

Les matériaux sol-gel nanoporeux sont notamment caractérisés en ce qu'ils présentent une surface spécifique d'adsorption de 15 ± 2 à 900 ± 100 m².g⁻¹, de préférence 150 ± 20 m².g-1 à 900 ± 100 m².g⁻¹. De manière avantageuse, les matériaux sol-gel microporeux présentent également une surface spécifique d'adsorption de 500 ± 50 m².g⁻¹ à 900 ± 100 m².g⁻¹, de préférence de 650 ± 70 m².g⁻¹ à 900 ± 100 m².g⁻¹ et plus préférentiellement encore de 750 ± 70 m².g⁻¹ à 900 ± 100 m².g⁻¹. Les matériaux sol-gel mésoporeux présentent avantageusement une surface spécifique d'adsorption de 15 ± 2 à 400 ± 40 m².g⁻¹ et plus préférentiellement encore de 150 ± 20 m².g⁻¹ à 300 ± 50 m².g⁻¹. La surface spécifique et la distribution des tailles de pores sont déterminées par analyse de l'isotherme d'adsorption-désorption à l'azote liquide avec le modèle DFT (Théorie de la Fonctionnelle de la Densité).

Ainsi le matériau permet d'éliminer les polluants nocifs tels que le formaldéhyde, molécule que les matériaux adsorbants classiques ne peuvent pas ou peu éliminer.

Dans un mode de réalisation, le matériau adsorbant spécifique peut comprendre une matrice nanoporeuse sol-gel d'oxydes métalliques, ladite matrice contenant au moins une molécule sonde portant au moins une fonction réactive pouvant réagir avec une fonction aldéhyde.

Les performances de dépollution/filtration de la cartouche sont déterminées notamment par les paramètres suivants :
- la proportion en masse entre le matériau adsorbant ou adsorbant classique et le matériau adsorbant spécifique,
- la surface spécifique du matériau adsorbant spécifique,
- la forme du matériau adsorbant spécifique,
- les caractéristiques physiques et chimiques du matériau adsorbant ou adsorbant classique.

Les exemples suivants illustrent l'invention sans en limiter la portée.

### EXEMPLES

### Exemple 1 : Matrice TMOS dopé dihydrazide d'acide adipique et acide sulfamique (Formulation E1).

**Réactifs** : Dihydrazide d'acide adipique (Numéro CAS 1071-93-8, masse molaire = 174.2 g.mol⁻¹, pureté 98%), TMOS (Numéro CAS : 681-84-5, masse molaire =152.2 g.mol⁻¹, densité d = 1.023 mg.cm⁻³), acide sulfamique (numéro CAS 5329-14-6, masse molaire = 97.1 g g.mol⁻¹, pureté grade technique). Moule en plastique en nid d'abeilles avec des puits cylindriques de 6 mm de diamètre et de 10 mm de profondeur.
V(TMOS) = 1463 mL
V(H₂O) = 2927 mL
dihydrazide d'acide adipique = 90.5 g (0.12M)
acide sulfamique = 51.2 g (0.12M)
V(total) = 4390 mL

**Mode opératoire :** 90.5 g de dihydrazide d'acide adipique et 51.2g d'acide sulfamique sont introduits dans un flacon de 5000 mL et 2927mL d'eau sont rajoutés. Le mélange est agité jusqu'à dissolution totale du dihydrazide d'acide adipique et de l'acide sulfamique. La solution aqueuse est introduite dans un réacteur double enveloppe de 5L thermostaté à 25°C. 1463 mL de TMOS sont rajoutés. Le mélange est laissé sous agitation mécanique. On observe la formation de 2 phases. Le mélange est laissé sous agitation jusqu'à ce que le mélange devienne homogène (10 min). Le mélange est versé dans un tiroir en PTFE équipé de moule en nid d'abeilles. Le tiroir est placé dans une enceinte de séchage thermostatée à 40°C. Le tiroir est balayé avec un flux de diazote (N₂) avec un débit de 10 L/min. Le séchage est arrêté après un ou plusieurs jours.

Les granulés secs obtenus sont translucides. Ces granulés sont recuits à 70°C pendant 4 heures.

Dans le présent exemple, les proportions molaires des réactifs sont TMOS/H₂O/dihydrazide d'acide adipique/acide sulfamique sont égales à 1/16.5/0.052/0.054.

### Exemple 2 : Matrice TMOS dopé dihydrazide d'acide adipique et acide sulfamique (Formulation E2).

**Réactifs** : Dihydrazide d'acide adipique (Numéro CAS 1071-93-8, masse molaire = 174.2 g.mol⁻¹, pureté 98%), TMOS (Numéro CAS : 681-84-5, masse molaire =152.2 g.mol⁻¹, densité d = 1.023 mg.cm⁻³), acide sulfamique (numéro CAS 5329-14-6, masse molaire = 97.1 g g.mol⁻¹, pureté grade technique). Moule en plastique en nid d'abeilles avec des puits cylindriques de 6 mm de diamètre et de 10 mm de profondeur.
V(TMOS) = 1463 mL
V(H₂O) = 2927 mL
dihydrazide d'acide adipique = 30.2 g (0.04M)
acide sulfamique = 17.1 g (0.04M)
V(total) = 4390 mL

**Mode opératoire** : 30.2 g de dihydrazide d'acide adipique et 17.1 g d'acide sulfamique sont introduits dans un flacon de 5000 mL et 2927mL d'eau sont rajoutés. Le mélange est agité jusqu'à dissolution totale du dihydrazide d'acide adipique et de l'acide sulfamique. La solution aqueuse est introduite dans un réacteur double enveloppe de 5L thermostaté à 25°C. 1463 mL de TMOS sont rajoutés. Le mélange est laissé sous agitation mécanique. On observe la formation de 2 phases. Le mélange est laissé sous agitation jusqu'à ce que le mélange devienne homogène (10 min). Le mélange est versé dans un tiroir en PTFE équipé de moule en nid d'abeilles. Le tiroir est placé dans une enceinte de séchage thermostatée à 40°C. Le tiroir est balayé avec un flux de diazote (N₂) avec un débit de 10 L/min. Le séchage est arrêté après un ou plusieurs jours.

Les granulés secs obtenus sont translucides. Ces granulés sont recuits à 70°C pendant 24H.

Dans le présent exemple, les proportions molaires des réactifs sont TMOS/H₂O/dihydrazide d'acide adipique/acide sulfamique sont égales à 1/16.5/0.017/0.018.

### Exemple 3 : matrice TMOS dopé dihydrazide d'acide adipique et acide phosphorique (Formulation E3).

**Réactifs** : Dihydrazide d'acide adipique (Numéro CAS 1071-93-8, masse molaire = 174.2 g.mol⁻¹, pureté 98%), TMOS (Numéro CAS : 681-84-5, masse molaire =152.2 g.mol⁻¹, densité d = 1.023 mg.cm⁻³), acide phosphorique (numéro CAS 7664-38-2, masse molaire = 98 g.mol⁻¹, pureté 85%). Moule en plastique en nid d'abeilles avec des puits cylindriques de 6 mm de diamètre et de 10 mm de profondeur.
V(TMOS) = 1446 mL
V(H₂O) = 2909 mL
dihydrazide d'acide adipique = 90.5 g (0.12M)
acide phosphorique = 35.52mL (0.12M)
V(total) = 4390 mL

**Mode opératoire** : 90.5 g de dihydrazide d'acide adipique et 35.52mL d'acide phosphorique sont introduits dans un flacon de 5000 mL et 2909mL d'eau sont rajoutés. Le mélange est agité jusqu'à dissolution totale du dihydrazide d'acide adipique et de l'acide sulfamique. La solution aqueuse est introduite dans un réacteur double enveloppe de 5L thermostaté à 25°C. 1446 mL de TMOS sont rajoutés. Le mélange est laissé sous agitation mécanique. On observe la formation de 2 phases. Le mélange est laissé sous agitation jusqu'à ce que le mélange devienne homogène (10 min). Le mélange est versé dans un tiroir en PTFE équipé de moule en nid d'abeilles. Le tiroir est placé dans une enceinte de séchage thermostatée à 40°C. Le tiroir est balayé avec un flux de diazote (N₂) avec un débit de 10 L/min. Le séchage est arrêté après un ou plusieurs jours.

Les granulés secs obtenus sont translucides. Ces granulés sont recuits à 70°C pendant 24H.

Dans le présent exemple, les proportions molaires des réactifs sont TMOS/H₂O/dihydrazide d'acide adipique/acide phosphorique sont égales à 1/16.5/0.052/0.054.

### Exemple 4 : Matrice TMOS/MTMS dopé dihydrazide d'acide adipique et acide sulfamique (Formulation E4).

**Réactifs** : Dihydrazide d'acide adipique (Numéro CAS 1071-93-8, masse molaire = 174.2 g.mol⁻¹, pureté 98%), TMOS (Numéro CAS : 681-84-5, masse molaire =152.2 g.mol⁻¹, densité d = 1.023 mg.cm⁻³), MTMS (Numéro CAS : 1185-55-3, Masse molaire =136.22 g.mol⁻¹, densité d = 0.955mg.cm⁻³), acide sulfamique (Numéro CAS 5329-14-6, masse molaire = 97.1 g g.mol⁻¹, pureté grade technique). Moule en plastique en nid d'abeilles avec des puits cylindriques de 6 mm de diamètre et de 10 mm de profondeur.
V(TMOS) = 1028 mL
V(MTMS) = 423 mL
V(H₂O) = 2939 mL
dihydrazide d'acide adipique = 90.5 g (0.12M)
acide sulfamique = 51.2 g (0.12M)
V(total) = 4390 mL

**Mode opératoire** : 90.5 g de dihydrazide d'acide adipique et 51.2g d'acide sulfamique sont introduits dans un flacon de 5000 mL et 2927mL d'eau sont rajoutés. Le mélange est agité jusqu'à dissolution totale du dihydrazide d'acide adipique et de l'acide sulfamique. La solution aqueuse est introduite dans un réacteur double enveloppe de 5L thermostaté à 25°C. 1028 mL de TMOS et 423 mL de MTMS sont rajoutés. Le mélange est laissé sous agitation mécanique. On observe la formation de 2 phases. Le mélange est laissé sous agitation jusqu'à ce que le mélange devienne homogène (10 min). Le mélange est versé dans un tiroir en PTFE équipé de moule en nid d'abeilles. Le tiroir est placé dans une enceinte de séchage thermostatée à 40°C. Le tiroir est balayé avec un flux de diazote (N₂) avec un débit de 10 L/min. Le séchage est arrêté après un ou plusieurs jours.

Les granulés secs obtenus sont translucides. Ces granulés sont recuits à 70°C pendant 24H.

Dans le présent exemple, les proportions molaires des réactifs sont TMOS/H₂O/MTMS/dihydrazide d'acide adipique/acide sulfamique sont égales à 1/16.5/0.3/0.052/0.054.

### Exemple 5 : Matrice TMOS dopé dihydrazide d'acide adipique et l'acide paratoluène sulfonique (Formulation E5).

**Réactifs** : Dihydrazide d'acide adipique (Numéro CAS 1071-93-8, masse molaire = 174.2 g.mol⁻¹, pureté 98%), TMOS (Numéro CAS : 681-84-5, masse molaire =152.2 g.mol⁻¹, densité d = 1.023 mg.cm⁻³), acide paratoluène sulfonique monohydraté (numéro CAS 6192-52-5, masse molaire = 190.2 g g.mol⁻¹, pureté ≥ 98%). Moule en plastique en nid d'abeilles avec des puits cylindriques de 6 mm de diamètre et de 10 mm de profondeur.
V(TMOS) = 1463 mL
V(H₂O) = 2927 mL
dihydrazide d'acide adipique = 45.25 g (0.06M)
acide paratoluène sulfonique monohydraté = 52.9 g (0.06M)
V(total) = 4390 mL

**Mode opératoire** : 90.5 g de dihydrazide d'acide adipique et 52.9 g d'acide paratoluène sulfonique monohydraté sont introduits dans un flacon de 5000 mL et 2927mL d'eau sont rajoutés. Le mélange est agité jusqu'à dissolution totale du dihydrazide d'acide adipique et de l'acide paratoluène sulfonique monohydraté. La solution aqueuse est introduite dans un réacteur double enveloppe de 5L thermostaté à 25°C. 1463 mL de TMOS sont rajoutés. Le mélange est laissé sous agitation mécanique. On observe la formation de 2 phases. Le mélange est laissé sous agitation jusqu'à ce que le mélange devienne homogène (10 min). Le mélange est versé dans un tiroir en PTFE équipé de moule en nid d'abeilles. Le tiroir est placé dans une enceinte de séchage thermostatée à 40°C. Le tiroir est balayé avec un flux de diazote (N₂) avec un débit de 10 L/min. Le séchage est arrêté après un ou plusieurs jours.

Les granulés secs obtenus sont translucides. Ces granulés sont recuits à 70°C pendant 24H.

Dans le présent exemple, les proportions molaires des réactifs sont TMOS/H₂O/dihydrazide d'acide adipique/ acide paratoluène sulfonique sont égales à 1/16.5/0.026/0.027.

### Exemple 6 : Matrice TMOS dopé dihydrazide d'acide téréphtalique et acide sulfamique (Formulation E6).

**Réactifs** : Dihydrazide d'acide téréphtalique (Numéro CAS 136-64-1, masse molaire = 194.194 g.mol⁻¹), TMOS (Numéro CAS : 681-84-5, masse molaire = 152.2 g.mol⁻¹, densité d = 1.023 mg.cm⁻³), acide sulfamique (Numéro CAS 5329-14-6, masse molaire = 97.1 g g.mol⁻¹, pureté grade technique). Moule en plastique en nid d'abeilles avec des puits cylindriques de 6 mm de diamètre et de 10 mm de profondeur.
V(TMOS) = 1463 mL
V(H₂O) = 2927 mL
dihydrazide téréphtalique = 25.58 g (0.03M)
acide sulfamique = 25.58 g (0.06M)
V(total) = 4390 mL

**Mode opératoire** : 25.58 g de dihydrazide d'acide téréphtalique et 25.58 g d'acide sulfamique sont introduits dans un flacon de 5000 mL et 2927mL d'eau sont rajoutés. Le mélange est agité jusqu'à dissolution totale du dihydrazide d'acide téréphtalique et de l'acide sulfamique. La solution aqueuse est introduite dans un réacteur double enveloppe de 5L thermostaté à 25°C. 1463 mL de TMOS sont rajoutés. Le mélange est laissé sous agitation mécanique. On observe la formation de 2 phases. Le mélange est laissé sous agitation jusqu'à ce que le mélange devienne homogène (10 min). Le mélange est versé dans un tiroir en PTFE équipé de moule en nid d'abeilles. Le tiroir est placé dans une enceinte de séchage thermostatée à 40°C. Le tiroir est balayé avec un flux de diazote (N₂) avec un débit de 10 L/min. Le séchage est arrêté après un ou plusieurs jours.

Les granulés secs obtenus sont translucides. Ces granulés sont recuits à 70°C pendant 24H.

Dans le présent exemple, les proportions molaires des réactifs sont TMOS/H₂O/dihydrazide d'acide téréphtalique/acide sulfamique sont égales à 1/16.5/0.013/0.027.

### Exemple 7 : Matrice TMOS dopé dihydrazide d'acide sébacique et acide sulfamique (Formulation E7).

**Réactifs** : Dihydrazide d'acide sébacique (Numéro CAS 925-83-7, masse molaire = 230.31 g.mol⁻¹), TMOS (Numéro CAS : 681-84-5, masse molaire = 152.2 g.mol⁻¹, densité d = 1.023 mg.cm⁻³), acide sulfamique (Numéro CAS 5329-14-6, masse molaire = 97.1 g g.mol⁻¹, pureté grade technique). Moule en plastique en nid d'abeilles avec des puits cylindriques de 6 mm de diamètre et de 10 mm de profondeur.
V(TMOS) = 1463 mL
V(H₂O) = 2927 mL
dihydrazide d'acide sébacique = 30.33 g (0.03M)
acide sulfamique = 25.58 g (0.06M)
V(total) = 4390 mL

**Mode opératoire** : 30.33 g de dihydrazide d'acide sébacique et 25.58 g d'acide sulfamique sont introduits dans un flacon de 5000 mL et 2927mL d'eau sont rajoutés. Le mélange est agité jusqu'à dissolution totale du dihydrazide d'acide sébacique et de l'acide sulfamique. La solution aqueuse est introduite dans un réacteur double enveloppe de 5L thermostaté à 25°C. 1463 mL de TMOS sont rajoutés. Le mélange est laissé sous agitation mécanique. On observe la formation de 2 phases. Le mélange est laissé sous agitation jusqu'à ce que le mélange devienne homogène (10 min). Le mélange est versé dans un tiroir en PTFE équipé de moule en nid d'abeilles. Le tiroir est placé dans une enceinte de séchage thermostatée à 40°C. Le tiroir est balayé avec un flux de diazote (N₂) avec un débit de 10 L/min. Le séchage est arrêté après un ou plusieurs jours.

Les granulés secs obtenus sont translucides. Ces granulés sont recuits à 70°C pendant 24H.

Dans le présent exemple, les proportions molaires des réactifs sont TMOS/H₂O/dihydrazide d'acide sébacique /acide sulfamique sont égales à 1/16.5/0.013/0.027.

### Mesure de la capacité de piégeage des formulations E1 à E7.

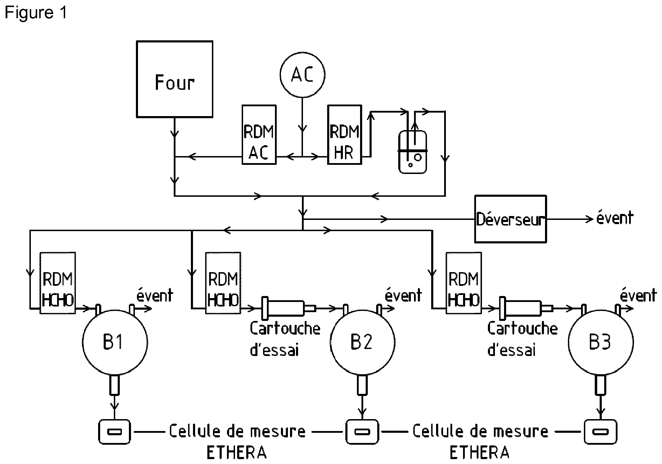

Les matériaux à tester ont été exposés à des concentrations de formaldéhyde de l'ordre de 400 ppbv à un débit de 4 l/min. Ces conditions ont été obtenues à l'aide du banc de génération ETHERA présenté en Figure 1.

Le formaldéhyde est généré dans un four à perméation régulé en température et en débit d'azote. Le formaldéhyde obtenu à des teneurs de l'ordre de plusieurs ppmv est dilué par flux d'air comprimé sec et un autre d'air comprimé humide (HR ≈ 100%) dans le but d'obtenir un flux d'air calibré à 400 ppbv de formaldéhyde et 50% d'humidité. Ce flux gazeux est ensuite séparé en trois et envoyé vers les ballons de prélèvement B1, B2 et B3. Sur les lignes menant à B2 et B3 se trouvent des cartouches d'essais contenant les matériaux à étudier. Le gaz pollué en formaldéhyde traverse donc les cartouches avant d'arriver dans les ballons B2 et B3.

Une à deux fois par jour un prélèvement gazeux est réalisé dans chacun des ballons afin de connaitre les concentrations en formaldéhyde traversant chacun d'eux. La valeur obtenue pour B1 nous donne la concentration en formaldéhyde en amont des cartouches d'essai tandis que les valeurs obtenues pour B2 et B3 nous donnent les concentrations en aval des cartouches d'essais. Les concentrations en formaldéhyde sont déterminée via la méthode du kit Profil'air Dynamic .

Pour chaque point de mesure on détermine la concentration moyenne en amont et en aval des cartouches et on converti ces concentrations en masse de formaldéhyde par minute qui arrive en amont des cartouches et qui ressort en aval. La différence entre ces deux valeurs permet de déterminer la masse de formaldéhyde piégée par minute d'exposition.

Pour calculer la quantité de formaldéhyde piégée, on suppose entre chaque point de mesure une évolution linéaire de la quantité piégée en fonction du temps. On trace la droite correspondante entre les deux points et on détermine l'équation de celle-ci. On intègre ensuite cette équation entre les deux points de mesure afin de déterminer l'aire sous la droite et donc la quantité de formaldéhyde piégée entre les deux points de mesures. La somme des aires ainsi déterminées représente la quantité totale de formaldéhyde piégée pendant la durée de l'essai.

Pour chaque matériau sol-gel poreux obtenus, formulations E1 à E7, la capacité de piégeage a été déterminée :

| Formulation | Capacité de piégeage (mg/g) |
|---|---|
| E1 | 11.1 |
| E2 | 3.2 |
| E3 | 7.0 |
| E4 | 6.5 |
| E5 | 5.3 |
| E6 | 3.1 |
| E7 | 7.2 |

## Revendications

1. Procédé de préparation d'un matériau sol-gel poreux adsorbant comprenant les étapes successives suivantes :
i) préparer une composition aqueuse comprenant au moins
- un précurseur sol-gel de type alcoxysilane ;
- un acide choisi parmi l'acide sulfamique, l'acide phosphorique, l'acide paratoluène sulfonique, l'acide parahydroxybenzoique et leurs mélanges ;
- une molécule sonde de formule générale (I) ou un de ses sels 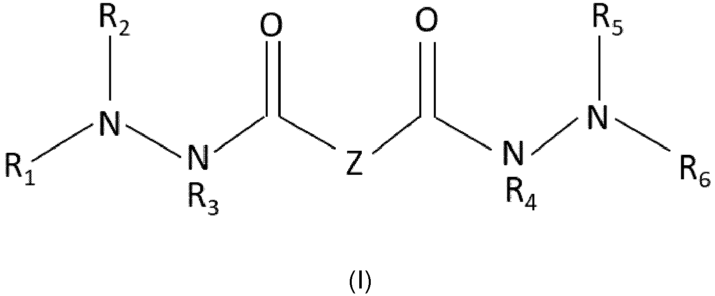
avec R₁, R₂, R₃, R₄, R₅, R₆ représentant indépendamment un atome d'hydrogène, un groupement (C1-C6)alkyle, un groupement (C3-C7)cycloalkyle ou un groupement alkyl-(C3-C7)cycloalkyle ;
avec Z représentant un groupement espaceur choisi parmi un groupement (C1-C16)alkyle, un groupement (C2-C16)alcényle, un groupement (C2-C16)alcynyle, un groupement (C1-C16)halogénoalkyle, un groupement aryle, un groupement aryloxy, un groupement carbocycle et un groupement aryl-(C1-C16)alkyle ;
ii) mettre en forme la composition obtenue à l'étape i) et appliquer un traitement thermique à une température supérieure à 20°C pour que la réaction d'hydrolyse / condensation se produise;
iii) sécher le matériau sol-gel adsorbant obtenu.

2. Procédé selon la revendication 1 **caractérisé en ce que** le précurseur de type alcoxysilane est choisi parmi l'orthosilicate de tétraméthyle (TMOS), le méthyltriméthoxysilane (MTMS), le tétraéthoxysilane (TEOS), le méthyltriéthoxysilane (MTES), le diméthyldiméthoxysilane et leurs mélanges, de préférence l'orthosilicate de tétraméthyle.

3. Procédé selon la revendication 1 **caractérisé en ce que** la molécule sonde ou son sel est le dihydrazide d'acide adipique (C₆H₁₄N₄O₂), le dihydrazide d'acide sébacique (C₁₀H₂₂N₄O₂) ou le dihydrazide de l'acide téréphtalique (C₈H₁₀N₄O₂).

4. Procédé selon la revendication 1 **caractérisé en ce que** la composition aqueuse mise en oeuvre à l'étape i) comprend un ratio molaire précurseur d'alcoxysilane / molécule sonde compris de 1/0,1 à 1/0,001, de préférence de 1/0,08 à 1/0,002, plus préférentiellement de 1/0,075 à 1/0,010, et en particulier un ratio égal à 1/0,05 ou 1/0,015.

5. Procédé selon la revendication 1 **caractérisé en ce qu'**il comprend une étape iv) de cuisson du matériau sol-gel.

## Patentansprüche

1. Verfahren zur Herstellung eines adsorbierenden, porösen Sol-Gel-Materials, umfassend die folgenden aufeinanderfolgenden Schritte:
i) Zubereiten einer wässrigen Zusammensetzung, umfassend mindestens
- einen Sol-Gel-Vorläufer vom Alkoxysilantyp;
- eine Säure, ausgewählt aus Amidosulfonsäure, Phosphorsäure, para-Toluolsulfonsäure, para-Hydroxybenzoesäure und deren Mischungen;
- ein Sondenmolekül der allgemeinen Formel (I) oder eines seiner Salze 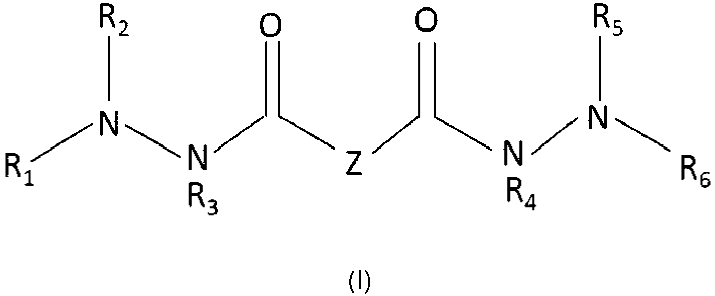
wobei R₁, R₂, R₃, R₄, R₅, R₆ unabhängig ein Wasserstoffatom, eine (C₁-C₆)-Alkylgruppe, eine (C₃-C₇)-Cycloalkylgruppe oder eine Alkyl-(C₃-C₇)-Cycloalkylgruppe darstellen;
wobei Z eine Abstandshaltergruppe darstellt, ausgewählt aus einer (C₁-C₁₆)-Alkylgruppe, einer (C₂-C₁₆)-Alkenylgruppe, einer (C₂-C₁₆)-Alkinylgruppe, einer (C₁-C₁₆)-Halogenalkylgruppe, einer Arylgruppe, einer Aryloxygruppe, einer Carbocyclusgruppe und einer Aryl-(C₁-C₁₆)-Alkylgruppe;
ii) Einbringen der in Schritt i) erhaltenen Zusammensetzung in eine Form und Anwenden einer thermischen Behandlung bei einer Temperatur über 20 °C, damit die Hydrolyse-/Kondensationsreaktion stattfindet;
iii) Trocknen des erhaltenen adsorbierenden Sol-Gel-Materials.

2. Verfahren nach Anspruch 1, **dadurch gekennzeichnet, dass** der Vorläufer vom Alkoxysilantyp ausgewählt ist aus Tetramethylorthosilikat (TMOS), Methyltrimethoxysilan (MTMS), Tetraethoxysilan (TEOS), Methyltriethoxysilan (MTES), Dimethyldimethoxysilan und deren Mischungen, vorzugsweise Tetramethylorthosilikat.

3. Verfahren nach Anspruch 1, **dadurch gekennzeichnet, dass** das Sondenmolekül oder sein Salz Adipinsäuredihydrazid (C₆H₁₄N₄O₂), Sebacinsäuredihydrazid (C₁₀H₂₂N₄O₂) oder Terephthalsäuredihydrazid (C₈H₁₀N₄O₂) ist.

4. Verfahren nach Anspruch 1, **dadurch gekennzeichnet, dass** die in Schritt i) realisierte wässrige Zusammensetzung ein molares Verhältnis von Alkoxysilanvorläufer : Sondenmolekül von 1:0,1 bis 1:0,001, vorzugsweise von 1:0,08 bis 1:0,002, bevorzugter von 1:0,075 bis 1:0,010 und insbesondere ein Verhältnis gleich 1:0,05 oder 1:0,015 umfasst.

5. Verfahren nach Anspruch 1, **dadurch gekennzeichnet, dass** es einen Schritt iv) des Brennens des Sol-Gel-Materials umfasst.

## Claims

1. Process for the preparation of a porous sol-gel adsorbent material, comprising the following successive steps:
i) preparing an aqueous composition comprising at least
- an alkoxysilane sol-gel precursor;
- an acid selected from sulphamic acid, phosphoric acid, paratoluenesulphonic acid, parahydroxybenzoic acid and mixtures thereof;
- a probe molecule of general formula (I) or a salt thereof 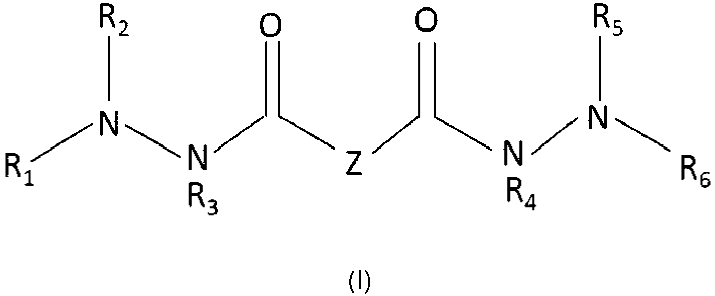
wherein R₁, R₂, R₃, R₄, R₅, R₆ independently represent a hydrogen atom, a (C₁-C₆)alkyl group, a (C₃- C₇)cycloalkyl group, or an alkyl-(C₃-C₇)cycloalkyl group,
wherein Z represents a spacer group selected from a (C₁-C₁₆)alkyl group, a (C₂-C₁₆)alkenyl group, a (C₂-C₁₆)alkynyl group, a (C₁-C₁₆)haloalkyl group, an aryl group, an aryloxy group, a carbocyclic group and an aryl-(C₁-C₁₆)alkyl group;
ii) shaping the composition obtained in step i) and applying heat treatment at a temperature above 20°C so that the hydrolysis/condensation reaction occurs;
iii) drying the sol-gel adsorbent material obtained.

2. Process according to claim 1, **characterised in that** the alkoxysilane precursor is selected from tetramethyl orthosilicate (TMOS), methyltrimethoxysilane (MTMS), tetraethoxysilane (TEOS), methyltriethoxysilane (MTES), dimethyldimethoxysilane and mixtures thereof, preferably tetramethyl orthosilicate.

3. Process according to claim 1, **characterised in that** the probe molecule or its salt is adipic acid dihydrazide (C₆H₁₄N₄O₂), sebacic acid dihydrazide (C₁₀H₂₂N₄O₂) or terephthalic acid dihydrazide (C₈H₁₀N₄O₂).

4. Process according to claim 1, **characterised in that** the aqueous composition used in step i) comprises a molar ratio of alkoxysilane precursor to probe molecule of from 1/0.1 to 1/0.001, preferably from 1/0.08 to 1/0.002, more preferably from 1/0.075 to 1/0.010, and in particular a ratio equal to 1/0.05 or 1/0.015.

5. Process according to claim 1, **characterised in that** it comprises a step iv) of baking the sol-gel material.
